# EUROPEAN PATENT APPLICATION

(11) **EP 1 410 722 A1**
(43) Date of publication of application: **21.04.2004**
(21) Application number: 02079289.1
(22) Date of filing: 16.10.2002
(51) Int. Cl.: A23L 1/29, A23L 1/305, A23L 1/308

(54) **Weight loss kit and method for losing weight**

(71) Applicant: Nutricia N.V., 2700 MA Zoetermeer (NL)
(72) Inventor: van Leare, Katrien Maria Jozefa, 6666 WS Heteren (NL); de Lange, Maria Elisabeth Hermien, 6701 DK Wageningen (NL)
(74) Representative: van Westenbrugge, Andries

(57) **Abstract**

There is provided a weight loss kit containing a first, a second and a third packaged edible composition, wherein the first edible composition contains caffeine, the second edible composition contains proteins and carbohydrates, and the third edible composition contains an appetite reducing ingredient.
Also provided herein is a method for the treatment or prevention of overweight in mammals, said method comprising sequentially administering to the human a diet comprising the first, the second and the third edible composition within a period of one day.

## Description

### FIELD OF THE INVENTION

The present invention relates to the maintenance of a healthy body weight, more particular to a weight loss kit and a method for the treatment and/or prevention of overweight, making use of said weight loss kit.

### BACKGROUND OF THE INVENTION

Obesity is a major health problem with approximately ninety-seven million people considered clinically overweight in the United States.

A vast variety of support aids are offered in the form of pharmaceutical compositions and nutritional supplements. However, the commercially available composition often do not provide the desired effect and have serious shortcomings. Hence, restriction of caloric intake appears to be an advantageous manner of reducing body weight or preventing the occurrence of overweight.

US-5595772 describes compositions and methods of losing weight in which an individual's daily caloric intake is limited to about 1600 calories or less, where the calories are provided by at least some form of carbohydrate intake and at least some form of protein intake, provided that about 40 percent or more of the protein intake is consumed at breakfast. The relative amounts of carbohydrate and protein ideally suited for each meal of the weight loss plan are described. Food packages include meals for at least one breakfast composition, at least one lunch or dinner composition, and at least one snack composition and, optionally may include, a traditional meal of a prescribed range of caloric and nutritional content to be consumed either at lunch or dinner.

The Scottsdale 7-Day Diet ™ is a meal plan including diet pills, appetite suppressing chews and meal replacement shakes. It is recommended to avoid caffeine while using the meal plan.

WO-96/39050 describes a dietary health management system for administration to a patient having at least one diet-responsive condition such as hypertension, hyperlipidemia, cancer, diabetes, and combinations thereof. The system comprises a plurality of prepackaged meals which may provide dietary fiber in the range of about 20 to 30 grams; vitamins and minerals at about 100 % USRDA; less than about 2400 mg of sodium; at least about 3500 mg of potassium; less than about 96 grams of simple sugars; protein, such that about 20 to 30 % of caloric intake is derived from protein; and fat, such that about 15 to 20 % of caloric intake is derived from fat.

Nowadays, a wide variety of nutritional products are advertised to reduce body weight. Nutritional supplements in the form of for example pills, drinks, bars and many other product forms are commercially available. However, a problem encountered with the use of these products in their individual form is that they often do not sort a sufficient effect. A main reason for the above problem is that the consumer appears to lack the ability to properly combine products and design an effective weight loss regimen.

To remove the burden of selecting a proper combination of nutritional products from the vast variety of nutritional compositions advertised to reduce body weight, several weight loss packages are commercially available. These are often referred to as "meal plan". The term "meal plan" as used in the present invention refers to a plurality of foodstuffs given in a predetermined order and at a predetermined time, said meal plan being suitably used in a method for the prevention and/or treatment of overweight. Meal plans are often provided in the form of a weight loss kit. The term "weight loss kit" as used in the present invention refers to a plurality of packaged edible compositions with instructions that direct the user to how to use each individual edible composition. The packaged edible compositions of the weight loss kit may be packaged together or presented separately with unifying indicia placed on the packaging. It was found by the present inventors that the presently available meal plans are not optimally designed for use in a method for the prevention and/or treatment of overweight, i.e. do not comprise optimal combination nutritional products or lack an optimal administration regimen. Hence, the meal plans do not result in the desired body weight reduction. A further cause of the limited effectiveness of known meal plans is the very low compliance to the available meal plans.

Accordingly, it is an object of the present invention to overcome the above problems. It is a further object of the present invention to provide a balanced and effective combination of edible compositions in the form of a weight loss kit and method for the prevention or treatment of overweight with said weight loss kit.

The present inventors have found that the main causes for the lack of compliance to a meal plan are:
- Replacement of two or more meals: Replacement of two or more meals selected from breakfast, lunch and dinner results in non-compliance to the meal plan. A strong appetite rapidly occurs due to the insufficient caloric intake, which is a major cause of non-compliance, particularly in the starting period of the meal plan.
- Replacement of breakfast and/or dinner: Breakfast and dinner have an important social value. Families sit together for some time while eating breakfast or dinner. If one of these meals is replaced by a shake or bar that is consumed within a very short period of time, the person following the meal plan will sit empty handed, finding him or herself excluded from the social activity. Additionally, the person following the meal plan, when replacing one or both of breakfast and/or dinner, will experience remarks from the other participants to the meal. This will pose a significant psychological burden on the person wishing to lose weight. For many this is an unacceptable burden, and thus a major cause of non-compliance to the meal plan.
- Overeating during dinner: Meal plans that do not replace dinner and only provide dietary restriction during the day often result in overeating at dinner. The calories ingested during the dinner annul the benefits of daytime caloric restriction or may even have an adverse effect if more calories are ingested during dinner compared to a situation where no meal plan was followed.
- Feelings of weakness and reduced energy: Subjects often experience a feeling of weakness when following a meal plan. In an attempt to improve spirit, high caloric (e.g. sugar containing) foodstuff are ingested, resulting in a failure to lose weight.
- Bad palatability: Many of the presently available diet programs include products that have bad palatability. Bad palatability is a major cause of non-compliance to the meal plan.
The presently available meal plans have not considered one or more of the above aspects, which almost inevitably results in compliance problems.

The present inventors have duly considered the above shortcomings of the currently available meal plans and found a weight loss kit, meal plan and method for the treatment and/or prevention of overweight that ensures an improved compliance. Advantageously, the present invention does not replace dinner or breakfast, thus results in a reduced social and psychological burden; provides a stimulant in the morning to prevent loss of spirit during the day or after a day of dieting; prevents overeating during dinner through ingestion of a low caloric appetite reducing composition shortly before or during dinner; has good palatability, partly because the breakfast and dinner meals remain unchanged.

It has now been found that the administration of at least three edible compositions within a period of one day, wherein the first edible composition is ingested shortly before, during or shortly after breakfast and contains caffeine and has a low caloric value, the second edible composition is ingested around lunchtime with the aim to replace the lunchtime meal and the third edible composition shortly before or during dinner aims to prevent overeating during dinner and contains at least one appetite reducing ingredient, advantageously overcomes the above mentioned problems by reducing the occurrence of overweight.

Hence, the present weight loss kit, meal plan and related administration regimen ensures a limited influence on the social life, good compliance, prevents overeating of dinner and stimulates spirit.

### SUMMARY OF THE INVENTION

In light of the above desired need , it is an aspect of the present invention to provide a weight loss kit comprising:
(a) a first packaged edible composition containing between 1 and 1000 mg caffeine and having a caloric content between 0 and 100 kcal;
(b) a second packaged edible composition in the form of a bar, reconstitutable preparation or drink containing between 1 and 150 grams digestible carbohydrates, between 1 and 150 grams protein and having a caloric content between 50 and 600 kcal;
(c) a third packaged edible composition containing at least one low caloric appetite reducing ingredient and having a caloric content between 0 and 50 kcal;
(d) indicia to distinguish said first, second and third edible composition; and
(e) instructions which teach the sequential use of said first edible composition, second edible composition and third edible composition.

In a further aspect the present invention relates to a method for the treatment and/or prevention of overweight said method comprising sequentially administering to the human a diet comprising a first, a second and a third edible composition within a period of one day, wherein
(a) the first edible composition contains between 1 and 1000 mg caffeine and between 0 and 100 kcal;
(b) the second edible composition is provided in the form of a bar or drink that contains between 1 and 150 grams digestible carbohydrates, between 1 and 150 grams protein and having a caloric content between 50 and 600 kcal; and
(c) the third edible composition contains at least one low caloric appetite reducing ingredient and between 0 and 50 kcal;

### Definitions

To facilitate an understanding of the invention, a number of terms are defined below:

The term " ready-to-drink " as used herein refers to a packaged preparation that does not need mixing with a liquid prior to ingestion.
The term "reconstitutable preparation" as used herein refers to a preparation that needs addition of a suitable liquid prior to ingestion.

The term "meal plan" as used in the present invention refers to a plurality of edible compositions given in a predetermined order and at a predetermined time, said meal plan being suitably used in a method for the prevention and/or treatment of overweight. A meal plan contains at least 3 edible compositions, which are instructed for ingestion at a predetermined time.
The term "indigestible fermentable sweetener" refers to sweeteners, that are not or only partially digested in the intestine by the action of acids or digestive enzymes present in the human upper digestive tract (small intestine and stomach) and which are at least partially fermented by the human intestinal flora.
The term "overweight" as used in the present invention refers to a bodyweight that is above the desired bodyweight of a human subject or that of a pet or farm animal as defined by its owner. Human subjects who have a body mass index above 25 kg per m² most advantageously use the present method and weight loss kit.

### DETAILED DESCRIPTION OF THE INVENTION

### First edible composition

The first edible composition, which is an essential element of the present invention is advantageously consumed in the morning, preferably between 6 and 10 AM, more preferably shortly before, during or shortly after breakfast, i.e. in the period between 30 minutes before and 30 minutes after breakfast.
The first edible composition is a low caloric composition that contains between 0 and 100 kcal, preferably between 0 and 75 kcal, more preferably between 0 and 50 kcal, even more preferably between 0 and 10 kcal.
Caffeine is an important ingredient of the first composition. Indeed, it was found that the caffeine in the morning will both increase the spirit and induce lipolysis of the fats stored in the adipocyte, thereby reducing adipose tissue mass. The liberated fatty acids will be converted into energy during the day providing both additional energy and preventing the conversion of the fatty acids into stored body fat.
The first composition contains between 1 and 1000 mg caffeine, preferably between 2 and 500 mg caffeine, even more preferably between 10 and 400 mg caffeine, most preferably between 25 and 350 mg caffeine. The caffeine may be chemically manufactured or provided in the form of a plant preparation. Preferably, the first composition comprises caffeine containing plant material

The present method preferably comprises the administration of the first composition in liquid form. It was found that the ingesting of solid edible compositions in the morning, in particular if the foodstuff has a bad palatability, is a cause of non-compliance to a meal plan. Hence, the first edible composition is preferably present in the weight loss kit as a reconstitutable preparation or a ready-to drink formula. Still, the first composition may be provided in the form of a pill, tablet or capsule, since these generally also have an acceptable palatability.

Preferably the first composition has good palatability. Hence, in a preferred embodiment the first composition contains sweetener and/or flavor. The sweetener preferably is a low caloric sweetener, more preferably selected from the group consisting of indigestible oligosaccharides, polyols, aspartame, sucralose, acesulfame potassium, alitame, cyclamate, saccharin and mixtures thereof. The amount of low caloric sweetener present in the first composition will greatly depend on the type of sweetener used. Preferably the first composition has a sucrose equivalent value (SEV) of at least 0.5, preferably at least 1, even more preferably at least 1.5 SEV. Preferably the SEV is below 25, more preferably below 10, even more preferably below 5.
The first composition preferably has volume, e.g. either as ready-to-drink or after reconstitution, between 5 and 250 ml, more preferably between 10 and 150 ml, even more preferably between 25 and 100 ml. It was found that a limited volume of the removes the burden of ingesting large volumes in the morning, which is a cause on non-compliance to the meal plan.

### Second edible composition

The second edible composition, which is also an essential element of the present invention, aims to replace the lunchtime meal and is provided in the form of a bar, reconstitutable preparation or drink. The ingredients of the second composition aim to provide the body sufficient calories and satiation (satiety) to function normally up until dinner by high caloric ingredients, namely carbohydrates and proteins.

Accordingly, the second composition contains between 1 and 150 grams digestible carbohydrates, between 1 and 150 grams protein and has a caloric content of between 50 and 600 kcal.
According to a preferred embodiment, the second composition is provided in the form of a reconstituable powder or drink, and the present method for prevention and/or treatment of overweight comprises the administration of the second composition in the form of a liquid or a drink.

### Proteins in the second composition

Proteins are essential in human nutrition. The second edible composition therefore preferably contains between 1 and 150 grams, more preferably between 1.5 and 100 grams, even more preferably between 2 and 50 grams, most preferably between 5 and 25 grams protein. Advantageously, the protein is selected from the group consisting of whey protein, casein, milk protein, soy protein and mixtures thereof. The protein is preferably at least partly vegetable protein. A preferred vegetable protein for use herein is soy protein. Indeed soy protein reduces blood serum cholesterol levels. Many people suffering from overweight have high blood serum cholesterol levels. Hence, advantageously, the second composition contains soy protein. Preferably, the soy protein is selected from the group consisting of soy protein concentrate, soy protein isolate and mixtures thereof. The second edible composition preferably comprises between 1 and 30 grams soy protein.

### Digestible carbohydrates in the second composition

The second composition contains between 1 and 150 grams digestible carbohydrates, preferably between 1.5 and 100 g, more preferably between 2 and 50 grams, even more preferably between 2 and 35 g. The term digestible carbohydrates includes digestible polysaccharides and monosaccharides, i.e. carbohydrates which are digested by the intestinal acids and/or intestinal enzymes and/or are absorbed by the intestinal cells. The digestible carbohydrate is preferably selected from the group consisting of starch, maltodextrin, monosaccharide, sucrose, trehalose and mixtures thereof. A preferred monosaccharide is fructose. The inclusion of digestible polysaccharides such as starches and maltodextrins in the second composition advantageously contributes to the feeling of satiety. When monosaccharides are present, it is preferred that the second composition contains between 0.5 and 20 grams, more preferably between 1 and 10 grams monosaccharide.

Digestible maltodextrins (DE between 5 and 30, preferably around 20) are advantageously added to the second composition to provide a satiety effect. Preferably, the second composition contains between 0.1 and 100 g maltodextrins, more preferably between 0.5 and 50 grams maltodextrins, even more preferably between 1 and 25 grams maltodextrins.

Optional ingredients can advantageously be added to the second composition. The optional ingredients are preferably selected from the group consisting of vitamin, indigestible fiber, mineral, fat, indigestible fermentable sweetener and mixtures thereof.

### Vitamins in the second composition

The second composition preferably comprises vitamins. The vitamins are suitably selected from the group consisting of Vitamin A, Vitamin E, Vitamin K, Vitamin C, Vitamin D, Vitamin B6, Folic acid, Vitamin B12, Thiamine, Riboflavin, Niacin, Choline, Biotin, Pantothenic acid and mixtures thereof. Preferably the second composition contains at least 3 vitamins selected from the above group. According to a preferred embodiment, at least vitamin D is present in the composition to stimulate the calcium absorption.
The vitamins are preferably present in the second composition in an amount of between 5% and 250% of the recommended daily administration (RDA) of that vitamin, preferably between 10 and 100 %, even more preferably between 20 and 90%. The RDA has been published by the US Food and Drug Administration.

### Fibers in the second composition

The second composition preferably includes one or more indigestible fibers selected from the group of soluble indigestible fibers, insoluble indigestible fibers and mixtures thereof. The fiber advantageously is capable of inducing a satiety effect by decreasing the rate of stomach emptying and/or provide a slow release of sugars in the intestine which also contributes to an extended feeling of satiety. Furthermore, the fibers may prevent or decrease constipation and/or contribute to a blood serum cholesterol lowering effect of the composition according to the present invention.
Preferably the fiber is included in the second composition in an amount between 0.1 g and 30 gram fiber, more preferably between 0.5 and 15 gram, even more preferably in an amount between 1 and 10 grams.
In a further preferred embodiment, the second composition contains water soluble indigestible fibre, preferably selected from the group consisting of pectin, alginate, chitosan, beta-glucan, soluble fibre from psyllium husk (hereafter referred to as psyllium), xanthan gum, guar gum, locust bean gum, gum arabic, soy fibre and mixtures thereof, more preferably from the group consisting of psyllium, pectin, alginate, beta-glucan and mixtures thereof.

In a preferred embodiment the second composition contains at least pectin and/or alginate. Pectin is divided into two main categories: high methoxylated pectin (hereafter referred to as HM pectin), which are characterized by a degree of methoxylation above 50% and low methoxylated pectin (hereafter referred to as LM pectin) having a degree of methoxylation below 50%. As used herein, "degree of methoxylation" (also referred to as DE or "degree of esterification") is intended to mean the extent to which free carboxylic acid groups contained in the polygalacturonic acid chain have been esterified (e.g. by methylation).

In a further preferred embodiment the second composition contains LM pectins. The LM pectin as used in the second composition preferably have a degree of methoxylation below 50%, preferably between 5% and 45%, more preferably between 10% and 40%, even more preferably between 15% and 35%.

The LM pectins in the second composition are capable of forming a sufficiently rigid matrix at a pH as present in the stomach of a normal human, e.g. pH 3. It is therefore preferred to include at least 0.15 g LM pectin, preferably at least 0.3 g LM pectin, more preferably at least 0.75 g, even more preferably at least 1.5 g, most preferably at least 2 g. Preferably the second composition has a LM pectin content below 5 wt.%, more preferably below 1.5 wt.% even more preferably below 1 wt.% based on the total weight of the second composition.

### Alginate

Alginates are linear unbranched polymers containing beta-(1→ 4)-linked D-mannuronic acid and alpha-(1→ 4)-linked L-guluronic acid residues with an average molecular weights (100 - 100000 residues) to suit the application. Suitable sources of alginate include seaweeds and bacterial alginates. Preferably sodium alginate and potassium alginate are used as a source of alginate.

The alginate in the present composition should be capable of forming a sufficiently rigid matrix at a pH as present in the stomach of a normal human, e.g. pH 3. It is therefore necessary to include at least 0.15 g, preferably at least 0.3 g, more preferably at least 0.75 g, even more preferably at least 1 g alginate. Preferably the present composition has an alginate content below 5 wt.%, more preferably below 2.5 wt.% even more preferably below 1 wt.% based on the total weight of the second composition.

### Minerals in the second composition

Preferably the second composition contains minerals. The minerals are suitably selected form the group consisting of calcium, iron, zinc, copper, chromium, iodine, selenium, magnesium, manganese, molybdenum, potassium, phosphate, chloride, sodium, phosphorus and mixtures thereof. Preferably the second composition contains at least 3 minerals selected from the above mentioned group. The second composition preferably contains between 5% and 250%, more preferably between 10 % and 100 %, most preferably between 10% and 50% of the recommended daily administration (RDA) of the specific mineral. The RDA has been published by the US Food and Drug Administration.

### Calcium in the second composition

The second composition advantageously comprises at least 10 mg calcium, more preferably at least 50 mg, even more preferably at least 100 mg, most preferably at least 250 mg. To maintain a good palatability, it is desirable that the second composition contains no more than 5 grams calcium, more preferably, no more than 2.5 grams calcium. Additional calcium reduces the risk of calcium deficiency. Calcium deficiency may result in the following symptoms: muscle cramps, brittle nails, eczema, aching joints, increased cholesterol levels, rheumatoid arthritis, tooth decay and numbness in the arms and/or legs. Additionally, calcium is of importance in compositions, which are used to prevent or treat overweight and obesity.

When the second composition contains pectin and/or alginate, it preferably contains a calcium salt, which is substantially less soluble in water at 20°C and at the pH of the composition then at 37°C and a pH below 5. Such a calcium salt, when present in the composition in an amount that exceeds its maximum solubility, will solubilize in the stomach under the influence of pH-reduction and/or temperature increase. Thus the calcium ion concentration in the composition will increase, which will automatically stimulate pectin and/or alginate gellation.

In a preferred embodiment, the calcium salt(s) used in the second composition has a solubility below 0.15 grams, more preferably below 0.1 grams, even more preferably below 0.06 grams per 100 ml (demineralised) water at 20°C and pH 7. The calcium salt is preferably selected from the group consisting of calcium phosphate (e.g. tribasic, dibasic, monobasic or penta calcium triphosphate), calcium carbonate, calcium sulfate, calcium oxide, calcium citrate (e.g. mono calcium citrate or tri calcium citrate), a calcium salt coated with a substance which has limited solubility in water at pH 7 and is soluble at a pH below about 5 (hereafter referred to as coated calcium salts) and mixtures thereof. Examples of coatings and methods for the preparations of coated calcium salts are given in WO-0038829, the entire content of which is hereby incorporated by reference. More preferably, the calcium salt is selected from the group consisting of coated calcium salt, calcium carbonate, calcium phosphate and mixtures thereof. Most preferably the majority of calcium salt is provided by calcium carbonate.

### Fat in the second composition

In a further preferred embodiment, the second composition includes lipids and/or fat. Preferably the lipids are obtained from plant sources, which have a relatively high content of monounsaturated and/or polyunsaturated fatty acids. Preferably at least about 10 wt.% of the lipids included in the second composition are polyunsaturated fatty acids. The content of lipids and/or fats included in the composition is preferably between 0.5 and 50 grams, more preferably between 1 and 15, most preferably between 1 and 10 grams.

### Indigestible fermentable sweetener in the second composition

In a further preferred embodiment the second composition comprises indigestible fermentable sweetener, particularly if the composition includes pectin and/or alginate. It was found by the present inventors that compositions providing a satiety effect through the gellation of low-methoxylated pectin and/or alginate significantly reduce calcium absorption and/or bioavailability. It was further found by the inventors that the calcium absorption inhibitory effect of fibers can be reduced by coadministration of fermentable sugars. Without wishing to be bound by theory, it is the inventors believe that the indigestible fermentable sweeteners stimulate the growth of the intestinal flora, thereby stimulating the degradation of fibers in the colon and liberating calcium. The liberated calcium can subsequently be absorbed. Preferably, the second composition includes indigestible fermentable sweetener, said sweetener being preferably selected from the group consisting of oligosaccharides, polyols and mixtures thereof.
Suitable oligosaccharides and their production methods are further described in Laere KJM (Laere, KJM, Degradation of structurally different non-digestible oligosaccharides by intestinal bacteria: glycosylhydrolases of Bi. adolescentis. PhD-thesis, Wageningen Agricultural University, Wageningen, The Netherlands) the entire content of which is hereby incorporated by reference.
According to a preferred embodiment the oligosaccharide in the second composition is selected from the group consisting of fructans, fructooligosaccharides, indigestible dextrins galactooligosaccharides (including transgalactooligosaccharides), xylooligosaccharides, soybean oligosaccharides, arabinooligosaccharides, glucooligosaccharides, mannooligosaccharides, fucooligosaccharides and mixtures thereof.

### Viscosity of the second composition

Ingestion of a meal replacer in solid form was found to reduce compliance to the mealplan, at least partly because meal replacement bars have reduced palatability. Hence, in a preferred embodiment the second composition is provided in the form of a drink or reconstitutable preparation, and is ingested in liquid form, i.e. ingested as a liquid having a viscosity below 600 mPas at a shear rate of 100s⁻¹. For this purpose, the second composition preferably has a viscosity below 600 mPas at a shear rate of 100s⁻¹ at 20°C and a viscosity of at least 125%, particularly at least 200% of said viscosity at a pH below 5 and a temperature of 37°C. Even more preferably, the second composition has a viscosity below 250 mPas, more preferably below 100 mPas, most preferably below 50 mPas at a shear rate of 100s⁻¹ at 20°C at around neutral pH. However, without wishing to be bound by theory, it is believed by the inventors that the ingestion of an edible composition with high viscosity induces satiety. Hence, in a particularly preferred embodiment the second composition increases in viscosity after ingestion (i.e. in the stomach). Suitable ingredients that will provide an increase in viscosity upon ingestion is are indigestible fibers. Such fibers will provide an increased viscosity of the composition at a pH below about 5. Preferred fibers for use in the second composition that do provide such increase viscosity are selected from pectin and/or alginate. These indigestible fibers enable the manufacture of a composition which has a greater viscosity under acidic pH compared to the viscosity at around neutral pH
Whenever the term viscosity used in the present document, this refers to the physical parameter which is determined according to the following method:
The viscosity may be determined using a Carri-Med CSL rheometer. The used geometry is of conical shape (6 cm 2 deg acrylic cone) and the gap between plate and geometry is set on 55 µm. A linear continuous ramp shear rate is used from 0 to 150 s⁻¹ in 20 seconds. The rheometer's thermostat is set on the appropriate temperature (e.g. 20°C or 37°C).
In order to determine the viscosity at acidic pH (pH 3), first a sufficient amount of 1 M HCl is homogeneously admixed (drop-wise under very gentle stirring for about 20 sec, to prevent the breakdown of the gel) to the liquid composition to adjust the pH of the composition to pH 3. Thereafter the composition is left standing at 20°C for about 10 minutes. Subsequently the composition thus obtained is used to determine the viscosity at pH 3, according to the method described above.

### Third edible composition

The third edible composition, which is also an essential element of the present invention, aims to prevent overeating during dinner. This is especially important since the dinnertime meal often includes vast quantities of high caloric ingredients such as fats. Accordingly, the third edible composition contains at least one low caloric appetite reducing ingredient and the composition has a caloric content between 0 and 50 kcal.

Advantageously, the third composition is a low caloric drink that contains at least one low caloric appetite reducing ingredient. Ingesting the third composition shortly before dinner (i.e. not more than 1 hour, preferably not more than 30 minutes before dinner) and/or during the dinner will reduce the appetite, preferably by providing a feeling of fullness and thereby preventing overeating at dinner; and/or reduce the absorption of high caloric components for the meal.
The low caloric appetite reducing ingredient preferably has an energy value below 3 kcal per gram, more preferably below 2 kcal per gram, even more preferably below 0.5 kcal per gram. In a particularly preferred embodiment, the low caloric appetite reducing ingredient has an energy value with is about 0 kcal/g. Advantageously the low caloric appetite reducing ingredient is water soluble. This solubility preferably exceeds 1 gram per liter, preferably exceeds 5 grams per liter water, even more preferably exceeds 25 g/l.
Preferably the third composition contains at least 80 wt.%, more preferably at least 90 wt.%, even more preferably at least 95 wt.% water based on the total weight of the third composition.
According to a preferred embodiment, the low caloric appetite reducing ingredient is selected from the group consisting of hydroxycitric acid, procyanidin, platycodin D, indigestible fiber and mixtures thereof. Preferably the third composition contains between 0.01 and 20 grams, preferably between 0.1 and 10 grams, more preferably between 0.5 and 7 grams indigestible fiber. Advantageously the third composition comprises between 0.01 and 10 grams, preferably between 0.05 and 5 grams, even more preferably between 0.1 and 3 grams hydroxycitric acid. The weight of platycodon D in the third composition is preferably between 0.001 and 4 grams, preferably between 0.01 and 2 grams, even more preferably between 0.025 and 1 gram platycodin D. Preferably the third composition contains between 0.001 and 10 g, preferably between 0.01 and 5 grams, more preferably between 0.05 and 3 grams, even more preferably between 0.1 and 2.5 g procyanidin.

According to a particularly preferred embodiment the third composition includes at least indigestible fiber, preferably water-soluble indigestible fiber. The indigestible fiber is advantageously included in an amount between 0.01 and 25 grams, more preferably between 0.1 and 10 grams indigestible fiber. When ingesting the third composition shortly before and/or during the dinner, the low caloric appetite reducing ingredient will provide a feeling of fullness, thereby preventing overeating at dinner, or even reducing the quantity of food ingested during dinner.

It is of further importance that the third composition has only a low caloric content, i.e. between 0 and 50 kcal, more preferably between 0 and 40 kcal, even more preferably between 0 and 10 kcal. Suitable compositions for use as a third component in the present method have for example been described in U.S. patent number 6248390, the entire content of which is hereby incorporated by reference.

The third edible composition preferably has satiety inducing capabilities. Furthermore, it is important that the third composition is in a form, which can be easily consumed shortly before or during dinner, preferably during dinner. In a particular preferred embodiment the third composition is ingested as a liquid. Hence the third composition is preferably provided in the form of a liquid (i.e. ready-to-drink) or as a reconstitutable preparation.

Advantageously the third composition may comprise an ingredient capable of reducing absorption of high caloric nutrients in the duodenum. Ingesting of an ingredient capable of reducing nutrient absorption results in a decreased absorption of calories from the meal compared to a situation where the ingredient was not ingested. Preferably the ingredient is capable of reducing intestinal carbohydrate and/or fat absorption. Suitable ingredients capable of reducing intestinal absorption of nutrients may be selected from the group consisting of lipase inhibitors, triglyceride binding ingredients, carbohydrase inhibitors, carbohydrate absorption inhibitors and mixtures thereof, particularly from the group consisting of gymnemic acid, chitosan and mixtures thereof.

For ease of consumption, particularly during dinner, it is preferred that the third composition has a low viscosity. The viscosity of the third component used in the present method is preferably is below 100 mPas, more preferably below 50 mPas, even more preferably below 25 mPas at a shear rate of 100s⁻¹ and 20°C (measured according to method as described above).

Advantageously, the third composition may comprise at least 10 mg calcium, more preferably at least 50 mg, even more preferably at least 100 mg, most preferably at least 250 mg. The presence of calcium in the third composition contributes to a reduced risk of calcium deficiency. Calcium deficiency may result in the several symptoms as hereinbefore mentioned in the second composition. Additionally, it is of importance in compositions, which are used to prevent or treat overweight and obesity.

In a preferred embodiment, the calcium salt(s) used in the third composition has a solubility below 0.15, more preferably below 0.1 gram, even more preferably below 0.06 gram per 100 ml (demineralised) water at 20°C and pH 7. Preferably, the calcium salt(s) provide more than 0.2 gram dissolved calcium per 100 ml water at a pH below 5 and a temperature of 37°C, more preferably it provides more than 0.5 g/100 ml water under these conditions. The solubility of the calcium salt at pH below 5 and at 37°C is not bound to an upper limit, however, is suitably below 500 g of the salt per 100 ml water.

The calcium salt is preferably selected from the group consisting of calcium phosphate (e.g. tribasic, dibasic, monobasic or penta calcium triphosphate), calcium carbonate, calcium sulfate, calcium oxide, calcium citrate (e.g. mono-calcium citrate or tri-calcium citrate), a calcium salt coated with a substance which has limited solubility in water at pH 7 and is soluble at a pH below about 5 (hereafter referred to as coated calcium salts) and mixtures thereof. Examples of coatings and methods for the preparations of coated calcium salts are given in WO-0038829, the entire content of which is hereby incorporated by reference.
To maintain a good palatability, it is desirable that the third composition contains no more than 5 grams calcium, more preferably, no more than 2.5 grams calcium.

The present inventors found that a drink containing pectin and/or alginate and insoluble calcium can be advantageously used as a third composition in the present method. It was surprisingly found that the drink containing a combination of pectin and/or alginate and calcium has a low viscosity and but is still capable of inducing a feeling of satiety.

When ingested, the drink containing a combination of pectin and/or alginate and calcium, reaches the stomach where it forms a matrix under the acidic conditions of the stomach. Without wishing to be bound by theory, it is believed by the inventors that the formation of a matrix in the stomach increases the release of cholecystokinin (CCK) hormones, inducing the feeling of satiety. The induced satiety in turns prevents overeating during dinner.

Preferably, at pH below 5 (e.g. pH 3) and 37°C, the composition has a viscosity which is at least 125 % of the viscosity at near neutral pH, more preferably above 150%. According to a further preferred embodiment the liquid edible composition at pH 3 and at 37°C has a viscosity of at least twice the viscosity of the composition at pH 7 and 20°C, preferably at least trice.

To achieve the characteristics of low viscosity at neutral pH with increased viscosity at acidic pH, it is preferred that the third composition contains the combination of pectin and/or alginate and a calcium salt which is substantially insoluble at a neutral pH. In a preferred embodiment, the third composition contains between 0.01 and 2 wt.% of pectin and/or of alginate; and between 0.01 and 1 wt.% calcium.

In a particularly preferred embodiment, the third composition as used in the present method comprises a liquid edible composition with a pH of more than 6, a viscosity below 50 mPas at a shear rate of 100s⁻¹ and 20°C, and a viscosity of at least 125% of the aforementioned viscosity at a pH below 5 (e.g. pH 3) and a temperature of 37°C, the composition comprising:
a) between 0.01 and 2 wt.% of pectin and/or of alginate; and
b) between 0.01 and 1 wt.% calcium

The third composition preferably contains less than 3 wt.%, preferably less than 2 wt.%, even more preferably less than 1.5 wt.% pectin and/or alginate and preferably at least 0.05 wt.%, preferably at least 0.1 wt.%, more preferably at least 0.25 wt.%, even more preferably at least 0.5 wt.% pectin and/or alginate based on the total weight of the third composition.
Advantageously, the third composition contains between 0.5 and 10 grams, even more preferably between 1 and 7 grams pectin and/or alginate.

In a preferred embodiment the third composition further contains an indigestible sweetener, even more preferably an indigestible sweetener that can be fermented by the intestinal bacteria, particularly an indigestible water-soluble sweetener that can be fermented by intestinal bacteria. Preferably the sweetener is selected from the group consisting of indigestible oligosaccharides, polyols, aspartame, sucralose, acesulfame potassium, alitame, cyclamate, saccharin and mixtures thereof.

The term indigestible oligosaccharides as used in the present invention refers to saccharides which have a degree of polymerisation of monose units exceeding 2, more preferably exceeding 3, most preferably exceeding 4, which are not or only partially digested in the intestine by the action of acids or digestive enzymes present in the human upper digestive tract (small intestine and stomach) but which are fermented by the human intestinal flora. The degree of polymerisation of the oligosaccharide is below 60 monose units, preferably below 40, even more preferably below 20, most preferably below 10. The term monose units refers units having a closed ring structure, preferably hexose, e.g. the pyranose or furanose forms. Suitable compositions for use as the third composition have for example been described in WO-9959542, the entire content of which is hereby incorporated by reference.
The term polyols refers to sugar alcohols. Preferably the polyol selected from the group consisting of sorbitol, erythritol, maltitol, mannitol, isomalt and xylitol.

It is a further advantage that the third composition contains one or more polyols. Polyols reduce the viscosity of the third composition, particularly in the case where the composition contains pectin and/or alginate. Preferably the polyols are selected from the group consisting of sorbitol, maltitol, mannitol, erythritol and mixtures thereof. In a preferred embodiment, mannitol is used. Preferably the above-mentioned viscosity decreasing ingredients are added in an amount of 0.01 to 10 wt.%, more preferably between 0.1 and 5 wt.% based on the total weight of the third composition.

For ensuring a low viscosity, easy manufacturing process and/or a low caloric content of the drink, the present liquid composition preferably includes only a minor amount of other ingredients. Hence, preferably the present composition contains less that 1 wt.%; preferably less than 0.1 wt.%, more preferably less than 0.01 wt.% protein based on the total weight of the drink. Advantageously, the third edible composition contains less than 1 wt.%, preferably less than 0.1 wt.%, more preferably less than 0.01 wt.% triglycerides based on the total weight of the drink. In a further preferred embodiment the third edible composition contains less than 3 wt.%, preferably less than 1 wt.%, more preferably less than 0.1 wt.% digestible carbohydrates based on the total weight of the drink.

To ensure good compliance, the third composition preferably has a sucrose equivalent value (SEV) of at least 0.2, preferably at least 0.5, even more preferably at least 1 SEV. Preferably the SEV is below 25, more preferably below 10, even more preferably below 5.

According to a further preferred embodiment 95 wt.%, preferably 96.5 wt.%, even more preferably at least 98 wt.%, most preferably at least 99 wt.% of the third composition, at the time of ingestion, consists of water and at least one low caloric appetite reducing ingredient. The caloric density of the third composition, particularly after reconstitution or as ready-to-drink, is preferably below 1 kcal/ml, even more preferably below 0.5 kcal/ml, most preferably below 0.1 kcal/ml.

### Snack meals and/or supplementary aids

In a further preferred embodiment the present weight loss kit further comprises one or more snack meal, supplementary aid or both. The term "snack meal" as used in the present invention refers to a solid edible composition that has a caloric content between 10 and 500 kcal, preferably between 25 and 250 kcal. The snack meal predominantly aims to reduce craving urges and/or feelings of hunger, thereby increasing the compliance to the diet. In the present method, a snack meal is preferably administered between 2 PM and 5 PM.
The term "supplementary aids" as used in the present invention refers to an edible composition in the form of a pill, tablet or capsule that contains at least one bioactive ingredient, e.g. a nutritional supplement. The term bioactive ingredients refers to any ingredient for which there is at least an indication that it has a beneficial effect on the health effect of the human body.
For the purpose of the present invention, the supplementary aid preferably contains at least one antioxidant, preferably selected from the group of vitamin E, flavonoids, lecithin and mixtures thereof, and aims to reduce radical formation. It is believed by the inventors that prevention of radical formation reduces the potential harmful effects of body weight reduction. The supplementary aid is preferably ingested between 7 and 11 PM.

### Administration

The present method encompasses the administration of the first edible composition between 6 and 10 AM, i.e. at around breakfast; the second edible composition is between 11 AM and 2 PM, i.e. lunchtime; and the third edible composition is shortly before or during dinner. Hence, the weight loss kit is preferably accompanied with instructions which teach the sequential use of said first edible composition, second edible composition and third edible composition as a regimen in association with weight loss such that said first edible composition is instructed for ingestion at a time between 6 and 10 AM, said second edible composition is instructed for ingestion at a time between 11 AM and 2 PM and said third edible composition is instructed for ingestion shortly before or during dinner. The third composition is preferably ingested at a time between one hour prior to one meal and the time of the end of that meal, more preferably shortly before and/or during dinner.

The weight loss kit preferably comprises indicia to distinguish said first, second and third edible composition. The indicia are preferably selected from the group consisting of alphabetic indicia, numeric indicia, color indicia, graphic indicia, and combinations thereof. Each edible composition preferably comprises different indicia.

Furthermore, the weight loss kit preferably comprises instructions, which teach the sequential use of the first, second and third edible composition in one day. The instruction may be in the form of indicia such as alphabetic indicia, numeric indicia, color indicia, graphic indicia, and combinations thereof.

In a further preferred embodiment, the weight loss kit comprises instructions or indicia which teach the use of the first edible composition, second edible composition and third edible composition as a regimen in association with weight loss. The indicia or instruction may be in any form, such as language directing the user towards the intended use, e.g. by providing the product with a name which points the consumer towards the intended use, such as by naming the product "slim-kit", "low caloric diet plan", "be-lean". Also pictures or drawings may be used to direct the consumer towards the intended use, such as a slim figure or a balance.

Preferably the present method comprises the ingestion of the edible compositions in the weight loss kit for at least 3, preferably for at least 7, even more preferably for at least 25 consecutive days. Preferably the present method comprises the ingestion of the edible compositions in the weight loss kit for not more than 350 consecutive day, even more preferably not more than consecutive 150 days.

### Treatment and prevention of overweight

The present invention provides a method for reducing or preventing overweight, more preferably a method for the reduction of body fat. The term "overweight" as used in the present invention refers to a body weight that is above the desired body weight of a human subject or that of a pet or farm animal as defined by its owner. The present method is particularly suitable for humans. Human subjects who have a body mass index above 25 kg per m² most advantageously use the present method. Packaged foodstuffs, particularly nutritional supplements and dietary products, which have been provided with labels that explicitly or implicitly direct the consumer towards the use of said supplement or product in accordance with one or more of the above purposes, are advantageously used in accordance with the present invention.

The present weight loss kit advantageously provides less than 2000 kcal, more preferably less than 1000 kcal, even more preferably less than 750 kcal, most preferably less than 500 kcal per day. The limited caloric content of the present meal plan is important, since the overweight user generally continues consumption of breakfast and dinner. High caloric content of the present weight loss kit would counteract the desired aim, i.e. the treatment or prevention of overweight.

Advantageously, at least two, preferably at least the three of the first, second and third edible composition are ingested as homogeneous mixtures. It is believed by the inventors that providing edible compositions as non-homogeneous preparations, i.e. as a meal comprising foodstuffs with different mouth-feel (e.g. potatoes and meat) reduces the compliance to the present meal plan.

### Packaging

Preferably the present weight loss kit comprises packaging unifying said first, second and third edible compositions, optionally combined with indicia and instructions. The present weight loss kit may be provided in the form of a container containing at least the first, second and third edible composition. Alternatively, each edible composition of the present weight loss kit may be provided separately with packaging or instructions unifying the edible compositions of the present weight loss kit. From the packaging and/or instruction, it should be sufficiently clear for a consumer that the edible compositions are part of the same meal plan and are part of the same weight loss kit.

A unity of one first composition, one second composition and one third composition is hereinafter referred to as "daily diet". A single dosage of the first, second or third composition is hereinafter referred to as "unit dosage".

The present weight loss kit preferably comprises at least one daily diet. Preferably the weight loss kit contains between 2 and 10 daily diets. The first, second and third composition may be packages in any suitable form, for example per unit dosage (i.e. a dosage which is ingested within about 30 minutes) or in a container comprising multiple unit dosages. If a composition is packaged in a form containing multiple unit dosages, it is preferably accompanied with indicia which describe the method for separating one unit dosage from the bulk and/or portion identification aids such as measuring devices for determining portion size.

## Claims

1. Weight loss kit comprising a first, a second and a third edible composition for use in a therapeutic method, preferably a method for the treatment and/or prevention of overweight, said method comprising sequentially administering to the human a diet comprising the first, the second and the third edible composition within a period of one day, wherein
(a) the first edible composition contains between 1 and 1000 mg caffeine and has a caloric content between 0 and 100 kcal;
(b) the second edible composition is provided in the form of a bar or drink that contains between 1 and 150 grams digestible carbohydrates, between 1 and 150 grams protein, and has a caloric content between 50 and 600 kcal; and
(c) the third edible composition contains at least one low caloric appetite reducing ingredient and has a caloric content between 0 and 50 kcal.

2. Weight loss kit comprising:
(a) a first packaged edible composition containing between 1 and 1000 mg caffeine and has a caloric content between 0 and 100 kcal;
(b) a second packaged edible composition in the form of a bar, reconstitutable preparation or drink containing between 1 and 150 grams digestible carbohydrates, between 1 and 150 grams protein and has a caloric content between 50 and 600 kcal;
(c) a third packaged edible composition containing at least one low caloric appetite reducing ingredient and has a caloric content between 0 and 50 kcal;
(d) indicia to distinguish said first, second and third edible composition; and
(e) instructions which teach the sequential use of said first edible composition, second edible composition and third edible composition

3. Weight loss kit according to claim 2, comprising instructions which teach the sequential use of said first edible composition, second edible composition and third edible composition as a regimen in association with weight loss.

4. Weight loss kit according to claim 2, comprising packaging unifying said first, second and third edible compositions, indicia and instructions.

5. Weight loss kit according to claim 1, wherein the first edible composition is administered between 6 and 10 AM; the second edible composition is administered between 11 AM and 2 PM; and the third edible composition is administered shortly before or during dinner.

6. Weight loss kit according to claim 2, wherein said first edible composition is instructed for ingestion between 6 and 10 AM, said second edible composition is instructed for ingestion between 11 AM and 2 PM and said third edible composition is instructed for ingestion shortly before or during dinner.

7. Weight loss kit according to any one of the preceding claims, wherein the first edible composition is provided in the form of a drink or a reconstitutable preparation.

8. Weight loss kit according to any one of the preceding claims, wherein the first edible composition is provided in the form of a tablet, pill or capsule.

9. Weight loss kit according to any one of the preceding claims, wherein the second edible composition is provided in the form of a drink or a reconstitutable preparation.

10. Weight loss kit according to any one of the preceding claims, wherein the second edible composition contains between 0.01 and 20 g indigestible fiber.

11. Weight loss kit according to any one of the preceding claims, wherein the second edible composition contains between 1 and 50 grams soy protein.

12. Weight loss kit according to any one of the preceding claims, wherein the second edible composition comprises between 1 and 250 % of the daily recommended intake of a vitamin selected from the group consisting of Vitamin A, Vitamin E, Vitamin K, Vitamin C, Vitamin D, Vitamin B6, Folic acid, Vitamin B12, Biotin, Pantothenic acid and mixtures thereof.

13. Weight loss kit according to any one of the preceding claims, wherein the third edible composition contains at least one ingredient selected from the group consisting of between 0.01 and 20 grams indigestible fiber, between 0.01 and 10 grams hydroxycitric acid, between 0.001 and 4 grams of platycodin, between 0.001 and 10 grams procyanidin and mixtures thereof.

14. Weight loss kit according to any one of the preceding claims, wherein the third edible composition contains between 0.1 and 10 grams indigestible fiber.

15. Weight loss kit according to any one of the preceding claims, wherein the third edible composition is provided in the form of a beverage or reconstitutable preparation.

16. Weight loss kit according to any one of the preceding claims, wherein the third edible composition contains a water-soluble indigestible fiber.

17. Weight loss kit according to any one of the preceding claims, wherein the third edible composition contains between 0.5 and 10 grams pectin and between 5 mg and 2 grams calcium salt.

18. Weight loss kit according to any one of the preceding claims, further comprising a snack meal which is instructed for ingestion between 2 PM and 5 PM

19. A cosmetic method for improving an individual's appearance by losing weight, comprising administering to the human a diet comprising the first, the second and the third edible composition within a period of one day, wherein
a. the first edible composition contains between 1 and 1000 mg caffeine and has a caloric content between 0 and 100 kcal;
b. the second edible composition contains between 1 and 150 grams digestible carbohydrates, between 1 and 150 grams protein and has a caloric content between 50 and 600 kcal;
c. the third edible composition contains at least one low caloric appetite reducing ingredient and has a caloric content between 0 and 50 kcal; and
d. the first edible composition is administered between 6 and 10 AM; the second edible composition is administered between 11 AM and 2 PM; and the third edible composition is administered shortly before or during dinner.
